# EUROPEAN PATENT APPLICATION

(11) **EP 3 251 586 A1**
(43) Date of publication of application: **06.12.2017**
(21) Application number: 16172056.0
(22) Date of filing: 30.05.2016
(51) Int. Cl.: A61B 5/00, H01F 17/00

(54) **IMPLANTABLE ANALGOUE DEVICE AND SYSTEM COMPRISING SUCH DEVICE**

(71) Applicant: ETH Zurich, 8092 Zurich (CH); University of Zurich, 8006 Zurich (CH)
(72) Inventor: VÖRÖS, Janos, 8050 Zurich (CH); STAUFFER, Flurin, 8432 Wernetshausen (CH); TYBRANDT, Klas, 602 45 Norrköping (SE); SCHNEIDER, Marc, 3612 Steffisburg (CH); KESSLER, Thomas, 3073 Gümligen (CH); KUENG, Roland, 8633 Wolfhausen (CH); HENGSTELER, Julian, 8046 Zürich (CH); HAGANDER, Michael, 8003 Zürich (CH); STOLL, André, 8132 Egg (CH); TRÜEB, Camill, 8132 Egg (CH); SUJATA, Jean-Marc, 8953 Dietikon (CH); HOFFMANN, Felix, 67480 Edenkoben (DE); SCHUURMANS STEKHOVEN, Joy, 8008 Zürich (CH); QUACK, Josefine, 8038 Zürich (CH); ZILLY, Hannes, 8046 Zürich (CH); GOEDEJOHANN, Johannes, 8006 Zürich (CH)

(57) **Abstract**

Implantable analogue device (1)
- configured for passively determining parameters representative of physiological parameters of a part of a human or animal body and
- configured for passive wireless transmission of the determined parameters, wherein the implantable device (1) comprises a resonant circuit (10) comprising a coil (20) and a capacitor (30), and
wherein the resonant circuit (10) comprises a strain sensing section (15), and wherein the resonant circuit (10) is configured to change its resonant frequency depending on a strain applied to the strain sensing section (15).

Furthermore, a system (5) comprising the implantable analogue device (1).

## Description

The invention relates to the field of medical technology, especially to implantable analogue devices and systems comprising implantable analogue devices. In particular the invention relates to implantable strain sensors, transmission circuits and readout circuits for determining and transmitting parameters representative of physiological parameters of a part of a human or animal body.

Information on physiological parameters of e.g. organs, blood vessels or other body parts can be extremely useful to the doctor and even vital for the patient. Implantable sensors that in vivo monitor physiological parameter of a human or animal body can significantly improve the quality, rate and/or costs of monitoring of physiological parameters as well as the life quality of the patients.

For example, neurogenic lower urinary tract dysfunction (NLUTD) not only has a highly negative impact on the quality of life of the patient, but may progressively even lead to end stage renal failure. Namely, the lower urinary tract dysfunction can cause high storage pressures, which can lead to urinary reflux towards kidneys and thereby to severe damage. Therefore, it is a vital issue to monitor the storage pressure in the bladder. The current gold standard for bladder pressure monitoring is urodynamic investigation, wherein a transurethral or suprapubic catheter is placed into the bladder. However, this procedure not only requires time and effort, but is also unprecise, may cause urinary tract infections and is costly. Since storage pressure is the most important clinical parameter regarding the upper urinary tract there is a need for new non- or minimal invasive pressure and/or extension measurement systems allowing for continuous or at least semi-continuous monitoring of the bladder state over time.

In DAKURAH et al: "Implantable Bladder Sensors: A Methodological Review" (Int Neurourol J 2015;19:133-141) recent trends and approaches for implantable bladder sensors are reviewed and drift, biocompatibility, hermetic packing, communication, operating power, and incompatibility with popular medical imaging tools are described as the main limitations. Recent approaches use strain sensors based on changes in capacitance or resistance for measuring bladder volume or pressure sensors with wireless readout. Nevertheless, these proposed sensors have the drawbacks that a (complex) implantable circuit is used for measurement and communication to the electronics outside of the body, the sensor is not suitable for high deformations (>50% strain), the sensor is not suitable for permanent implantation, the sensor design is not applicable with minimally invasive implantation procedures, and/or that the material and used design requires complex encapsulation strategies.

The object of the invention is to provide an improved implantable device for measuring and transmission of physiological parameters, which realises at least one of the following:
- it is suited for high deformation, in particular to above 50% unilateral stretching,
- it has a simple circuit,
- it is suited for implantation without complex encapsulation strategies,
- it is suited for permanent implantation,
- it is suited for minimally invasive implantation procedures, and/or
- it is suited to be used in various areas, e.g. in regard to various organs, blood vessels or other body parts.

The object is achieved by an **implantable analogue device** and a **system** comprising an implantable analogue device according to the features of the respective claims. "Implantable" means that the device is configured to be implanted into a human and/or an animal body. "Analogue" means that the device comprises no digital component.

The invention relates to an implantable analogue device that is
- configured for determining parameters representative of physiological parameters of a part of a human or animal body, and
- configured for wireless transmission of the determined parameters.

The implantable analogue device comprises a resonant circuit comprising a coil and a capacitor. Such a resonant circuit is also called an LC-resonant circuit.
Furthermore, the resonant circuit comprises a strain sensing section, wherein the resonant circuit is configured to change its resonant frequency depending on a strain applied to the strain sensing section.

It should be understood that the phrase "comprises/comprising a" shall mean "comprises/comprising one or more". In particular,
- the implantable analogue device can comprise one or more resonant circuits,
- each resonant circuit can comprise one or more coils and one or more capacitors, and
- each resonant circuit can comprise one or more strain sensing sections.

It should be understood that a resonant circuit can have one or more resonant frequencies; and whenever the text refers to the resonant frequency of a circuit it can represent the set and/or a subset of all resonant frequencies of that circuit. An embodiment with multiple resonant frequencies can have the advantage of improved measuring precision.

In an embodiment the implantable analogue device is
- configured for passively determining parameters representative of physiological parameters of a part of a human or animal body,
   and is
- configured for passive wireless transmission of the determined parameters.

When the device is implanted to e.g. a bladder wall, the strain sensing section can determine a parameter - e.g. the strain - of the bladder wall, which e.g. is representative of the volume of the bladder. The volume of the bladder is representative of the storage pressure of the bladder. Therefore, the storage pressure of the bladder can be estimated - e.g. by using an individual calibration - from the strain applied to the strain sensing section. When strain is applied to the strain sensing section, e.g. because the bladder's storage pressure increases, the resonant frequency of the resonant circuit changes. The resonant frequency of a resonant circuit can wirelessly be transmitted, e.g. by reading out the resonant frequency using a second electronic circuit outside of the body.

In an embodiment, - at least for an interval of magnitude of strain applied in at least one direction - the resonant frequency (of resonant circuit of the implantable analogue device) is a function essentially monotonously depending on a uniaxial strain applied to the strain sensing section,
in particular wherein the interval is between 0% uniaxial strain and 100% uniaxial strain in the at least one direction.

In particular, the resonant frequency can be strictly monotonously depending on the uniaxial strain applied to the strain sensing section.
A strictly monotonous function can be inverted and thus the applied strain can be determined from the resonant frequency.
For many applications not the exact value of a parameter, but coarse information (e.g. an approximate estimate of a parameter and/or the leaving of a given parameter range) is sufficient. Such coarse information can be determined e.g. from an essentially monotonous function.

The resonant circuit can comprise two or more strain sensing sections. For example, a first strain sensing section being part of the capacitor and a second strain sensing section being part of the second implantable analogue device coil. The resonant frequency can depend on the strain applied to the one or more strain sensing sections.

In an embodiment all elements of the resonant circuit are strain sensitive.

In an embodiment,
- the coil is configured to change its inductance depending on a strain applied to the coil or parts thereof; and
- the resonant circuit is configured to change its resonant frequency depending on the change of the inductance of the coil.

Such an embodiment is e.g. realized if the coil comprises or is designed as a strain sensing section.

In an embodiment,
- the capacitor is configured to change its capacitance depending on a strain applied to the capacitor or parts thereof; and
- the resonant circuit is configured to change its resonant frequency depending on the change of the capacitance of the capacitor.

Such an embodiment is e.g. realized if the capacitor comprises or is designed as a strain sensing section.

In an embodiment, the electrically functional parts of the implantable analogue device form the resonant circuit.

In a minimalistic embodiment, the implantable analogue device is the resonant circuit and the resonant circuit consists solely of the coil and the capacitor.

In an embodiment, the resonant circuit further comprises a resistor,
- wherein the resistor is configured to change its impedance depending on a strain applied to the resistor or parts thereof, and
- wherein the resonant circuit is configured to change its quality factor (also called the Q-factor) depending on a change of the impedance of the resistor.

Such an embodiment is e.g. realized if the resistor comprises or is designed as a strain sensing section.

In an embodiment, the resonant circuit of the implantable analogue device comprises two or more coils and/or two or more capacitors.

In an embodiment a further part of the resonant circuit of the implantable analogue device, in particular an additional coil and/or capacitor, is connected to the rest of the circuit via a switch and/or resistor. Such a switch can be arranged to switch on or off depending on the strain applied to the switch and/or the environment thereof. Such a resistor can be arranged to change its resistance depending on the strain applied to the resistance and/or the environment thereof. Thereby, the influence of the further part of the circuit on the resonant frequency can be varied depending on the applied strain.

In an embodiment, the implantable analogue device, in particular the strain sensing section, comprises an **elastically stretchable material,**
- wherein the elastically stretchable material is - in at least one direction - elastically stretchable at least up to 10% strain, in particular up to 100%, 150% or 200% strain, and
- wherein the elastically stretchable material is - at least partially- electrically conductive for any strain in the at least one direction at least up to 10% strain, in particular up to 100%, 150% or 200% strain.

In an embodiment, the implantable analogue device comprises multiple strain sensing sections comprising of an elastically stretchable material,
- wherein the elastically stretchable material is - in at least one direction - elastically stretchable at least up to 10% strain, and
- wherein the elastically stretchable material is - at least partially- electrically conductive for any strain in the at least one direction at least up to 10% strain, and wherein the strain sensing sections are arranged so that the implantable analogue device is designed to measure any strain in the at least one direction at least up to 100% strain. Such an embodiment can e.g. comprise ten strain sensing sections, each - in at least a first direction - stretchable up to 10% and - at least partially- electrically conductive for any strain in at least the first direction up to 10% strain, wherein the ten strain sensing sections are arranged in succession in the first direction.

Certain body parts can expand massively. E.g. the bladder can expand up to eleven times its minimal volume. In an example, a uniaxial strain sensor for determining the bladder volume can be stretchable at least up to (11^{1/3} - 1)%, which is approximately 122%. However, the strain sensitivity and/or stretchability needed to measure a certain expansion can depend on the location and the characteristics (e.g. size) of the implantable analogue device. In the case of the bladder, it is also possible to measure large expansions (e.g. eleven times its minimal volume) using an implantable analogue device whose strain sensitivity and/or stretchability is low (e.g. much less than 122%).

"At least partially" here means that at least a part of the elastically stretchable material is electrically conductive and electrically connects a first terminal to second terminal.

"Conductive" here means the conductivity at a temperature of 293 K is at least 10⁴ S/m, in particular more than 10⁵ S/m.

In an embodiment, the Young's Modulus of the elastically stretchable material is close to that of the tissue, e.g. to that of the bladder wall.

The Young's Modulus of the elastically stretchable material can be lower than 10 MPa, in particular lower than 1MPa or 100kPa.

In an embodiment, the elastically stretchable material comprises
- a support element that is elastically stretchable and electrically insulating, and
- electrically conductive elements supported by the support element and configured to form electrically conductive tracks for any strain at least up to 100%, in particular at least up to 150% or up to 200%, in at least one direction of strain.

The support element can comprise a polymer matrix, in particular an elastomer, e.g. polydimethylsiloxane (PDMS) or polyurethane or fluorinated silicone.

The electrically conductive elements can comprise amorphous metallic alloys, liquid metals, non-metallic conductors, silver, gold, platinum, copper, nickel, iridium, titanium, niobium, tantalum, silicon, gallium, indium, indium-gallium, phosphides, carbon nanotubes and/or graphene.

The electrically conductive elements can be particles, wires, ribbons and/or sheets. For example, the electrically conductive elements are elongated wires arranged such that for a first strain in one direction some of the elongated wires overlap and have sufficient overlap in that one direction so that for a second strain in that direction the elongated wires still overlap.

In an embodiment, at least some, in particular more than 50% or essentially all or all, of the electrically conductive elements in at least one dimension are smaller than 10µm. That is to say the electrically conductive element can be circumscribed by a rectangular cuboid, wherein at least one of the sides of the cuboid is smaller than 10µm.

In an embodiment, the electrically conductive elements form a dynamic network of galvanic connections, in particular wherein the network of galvanic connections is configured so that the number and topology of the galvanic connections depends on the applied strain. For example, - for a first strain - a first set of electrically conductive elements can form a first set of galvanic connections, and - for a second strain - a second set of electrically conductive elements can form a second set of galvanic connections.

In an embodiment, galvanic connections between the electrically conductive elements open and close depending on the strain. The conductivity of a trail of electrically conductive elements between a first terminal and a second terminal as a function of applied strain can be a step function (a locally constant function), namely depending on which galvanic connections are opened or closed. From the theory of Riemannian Integration it is well known that any piecewise continuous function on a finite interval can be approximated by the superposition of step-functions. Such superposition can in practise be carried out by connecting multiple trails between the first and the second terminal in parallel. In particular, at least within an interval of magnitude of strain, the conductivity between a first terminal and a second terminal can be constant, linearly increasing or linearly decreasing.

More details on suitable composite materials are e.g. disclosed in MARTINEZ et al: "Stretchable Silver Nanowires - Elastomer Composite Microelectrodes with Tailored Electrical Properties" (ACS Appl. Mater. Interfaces 2015, 7, 13467-13475)

If e.g. a plate capacitor is made of an elastically stretchable material whose conductivity for a direction of strain is at least locally constant, and a first strain in that direction is applied,
- the area of at least one of the electrodes of the plate capacitor, and/or
- the thickness of at least one of the electrodes of the plate capacitor, and/or
- the distance between the two electrodes of the plate capacitor can be different compared to the respective parameter for a second strain. Such effect can e.g. arise because of the Poisson effect and/or pressure arising in the elastically stretchable material. A change in the geometry (e.g. area, thickness, distance) can cause a change in the capacitance of the capacitor and thereby change the resonant frequency of the resonant circuit.

In an embodiment, the conductivity of the electrically conductivity of the elastically stretchable material is a non-constant function, e.g. a strictly monotonous function of the strain, in at least one direction of strain. For example the resonant circuit comprises a resistor comprising such elastically stretchable material with a strictly monotonous conductivity function, whereby the impedance of the resistor varies depending of the strain applied to the resistor and the quality factor of the resonant circuit varies depending on the impedance of the resistor. Elastically stretchable materials with a non-constant conductivity function can also be comprised in strain sensing sections of other components of the resonant circuit for varying the quality factor depending on the applied strain.

In an embodiment,
- the coil or a part of the coil; and/or
- the capacitor or a part of the capacitor
are made of the elastically stretchable material.

In an embodiment, only the coil is made of the elastically stretchable material. In another embodiment, only the capacitor is made of the elastically stretchable material. Having only the coil or the capacitor made of the elastically stretchable material can improve the linearity of the device, improve the performance, increase the quality factor, enable further miniaturization and/or simplify the calibration.

In an embodiment, the resonant circuit is completely made of the elastically stretchable material, in particular wherein the implantable analogue device is completely made of the elastically stretchable material. The elastically stretchable material may comprise conductive and non-conductive components, e.g. a non-conductive support element and electrically conductive elements for forming conductive tracks in the elastically stretchable material.

In an embodiment, the coil comprises a ferrite core. A ferrite core can improve the inductance of the coil and thereby allow for the creation a smaller device.

In an embodiment, the coil is a planar coil, a meander coil, a foil coil and/or a ribbon coil. The diameter of the coil can be less than 3 cm. That is to say that every two points of the coil have a distance smaller than 3 cm.

In an embodiment, the coil is a solenoid coil or a spring coil. The length of the coil can be less than 2cm and the diameter of the profile of the coil can be less than 5 mm. That is to say that the solenoid coil can circumscribed by a right circular cylinder, wherein in the cylinder has a base whose diameter is less than 5 mm, and wherein the height of the cylinder is less than 2 cm.

In an embodiment, the inductance of the coil is between 100 nH and 1 mH.

In an embodiment, the capacitor is a radial capacitor, a parallel plate capacitor and/or a finger capacitor. In particular, the capacitor can be less than 1 cm wide and less than 2 cm long. That is to say that the capacitor can be circumscribed by a rectangular cuboid, wherein the length of a first side of the cuboid is less than 1cm and the length of a second side of the cuboid is less than 2 cm.

In an embodiment, the capacitance of the capacitor is between 100 fF and InF.

In an embodiment, the resonant frequency of the resonant circuit is between 100 KHZ to 1GHz, in particular between 1 MHz and 40 MHz, in particular between 6.78 MHz and 13.56 MHz.

In an embodiment, the resonant circuit comprises two or more capacitors, wherein
- a first capacitor has a fixed capacitance, and
- a second capacitor has a capacitance depending on the applied strain.

In particular, the first capacitor can be un-stretchable. The capacitor with the fixed capacitance can be used to tailor the resonant frequency to be in a desired frequency band. Furthermore, such an embodiment can be beneficial to calibration and/or long time stability of the implantable analogue device.

The implantable analogue device can be used for passively determining parameters representative of physiological parameters of various organs and body parts, e.g. of the heart, stomach, blood vessels, diaphragm, prostate, uterus, liver, kidneys, pancreas, gall, bladder, lung, eyes and/or brain.

In an embodiment, at least a surface of the implantable analogue device is made of bio-compatible material. For example the support element of an elastically stretchable material can be made of bio-compatible material.

In an embodiment, the implantable analogue device is configured to be implantable to the outside of the bladder wall, into the bladder wall and/or into the bladder. The implantable analogue device can be configured to measure
- a volume of the bladder, and/or
- an extension of the bladder, and/or
- a pressure of the bladder and/or
- a physiological parameter related to the volume, the extension and/or the pressure of the bladder,
if implanted to the outside of the bladder wall, into the bladder wall and/or into the bladder.

The implantable analogue device can be stitched or glued to (e.g. outside of the bladder) or into the bladder wall or into the bladder (i.e. inside of the bladder). There exist multiple options for placement of the implantable analogue device. In a first option, the implantable analogue device is attached inside the bladder wall. This placement is particularly useful for very small implantable analogue devices, which e.g. can be implanted using a syringe/cystoscope and this placement is likely to reduce an infection of the implanted device. In a second option, the implantable analogue device is attached to the outer surface of the bladder wall bladder wall, e.g. by open and/or laparoscopic surgery. A third option is the intermittent placement of the implantable analogue device in the bladder by cystoscopic application. This approach allows measurement for a specific timeframe. The sensor will then e.g. be automatically removed automatically during voiding by normal micturition after the measurements.

The invention furthermore relates to a **system** comprising
- an implantable analogue device - e.g. as described above - ; and
- a readout circuit for reading out the resonant frequency of the resonant circuit of the implantable analogue device.

The readout circuit can be coupled to the implantable analog device through the means of inductive coupling between
- a primary coil comprised in the readout circuit, and
- the coil of the resonant circuit of the implantable analogue device as a secondary coil.

The readout circuit and the resonant circuit of the implantable analogue device can form a loosely coupled transformer.

In an embodiment, the readout circuit is configured for a readout using a frequency sweep, namely
- the readout circuit emits a sweep signal in a pre-defined frequency band,
- the readout circuit measures the reflected voltage and/or standing wave ratio for each frequency in the sweep signal,
- the readout circuit determines the resonant frequency of the resonant circuit and/or the coupled resonant circuit and readout circuit based on local extreme points of the measured voltages and/or standing wave ratio.

The sweep signal is a signal whose frequency continuously changes from a first frequency to a second frequency. It can drive an electromagnetic field, in particular a purely magnetic field, by which the readout circuit and the implantable analogue device interact. The oscillation frequency can e.g. be measured using a network analyser.

The primary coil can be matched to a line impedance in order to measure forward and reverse traveling waves. In this case, the sweep signal is the forward wave voltage while the reflection due to the introduced mismatch of the sensor is seen as a reverse wave voltage. As the sensor's impedance varies with frequency, the reflected wave voltage also varies accordingly.

In an embodiment, the readout circuit is configured for a readout using excitation and decay, namely
- the readout circuit induces a current of a chosen frequency (e.g. 6.78 MHz or 13.56 MHz) into the resonant circuit, thereby storing energy in the resonant circuit, and
- the readout circuit stops inducing the current, and
- the readout circuit measures the oscillation frequency of the resonant circuit.

This method/embodiment makes use of the fact that a system - in the absence of external excitation - oscillates with its resonant frequency of the resonant circuit of the implantable analogue device. The oscillation frequency can e.g. be measured using a network analyser and/or a spectrum analyser and/or performing a Fast Fourier Transformation. The readout circuit can comprise one or more coils.

In an embodiment, the system comprises two or more implantable analogue devices. Such an embodiment can be useful for acquiring local or environmental information, which can be interesting for several medical purposes. E.g. a two or more implantable analogue devices attached to a bladder wall can not only gather information on the bladder itself but also on the neighbouring prostate.

The resonant frequency of the resonant circuit of a first implantable analogue device can be substantially different, e.g. differ by at least 1 MHz, from the resonant frequency of the resonant circuit of a second implantable analogue device. Thereby the resonant frequency of the resonant circuit of the first implantable analogue device can be read out independently of the second implantable analogue device and vice versa by using a frequency sweep.

In an embodiment, the system comprises a communication apparatus (e.g. a mobile phone, tablet or a smartwatch) or a consumer electronic apparatus (e.g. a MP3-Player or a gaming console). In particular, the readout circuit can be integrated into the communication apparatus or the consumer electronic apparatus.

The readout can be made using an input signal by a user. Where the readout circuit is integrated into a smartphone, the user e.g. can activate the readout using an app on the smartphone or a mechanical button at the smartphone.

The readout can be made continuously or at least semi-continuously. For example, where the readout circuit is carried close enough to the implanted analogue device, the readout circuit can automatically readout the resonant frequency periodically, e.g. every five minutes. If the determined parameter indicates that a physiological parameter is outside a defined parameter range, an apparatus connected to the readout circuit can indicate a warning message to the patient and/or medical staff. The parameter range could be determined abstractly and/or by calibration. E.g. if the implantable analogue device is implanted to a bladder, the filling level of the bladder can be determined using a conventional bladder catheter and matched with the strain parameter determined by the implantable analogue device, i.e. calibration can be performed during a conventional urodynamic measurement.

In an embodiment, the system comprises a software, wherein the software
- provides a graphical user interface (commonly abbreviated GUI) for patients and/or doctors, and/or
- is configured for data analysis, and/or
- is configured for graphical display of data.

The software can run e.g. on a smartphone which comprises the readout circuit or is connectable to the readout circuit. The software can be used to start measurements respectively readouts, to analyse development, maxima, minima, etc. The software can furthermore be used to input parameter ranges such that e.g. - if an automatically measured parameter is outside the defined parameter range - a warning signal warns the user.

In an embodiment, the system comprises an apparatus, in particular an implantable apparatus for unloading fluid from the bladder, e.g. induction of the micturition. The apparatus can comprise the implantable analogue device. Such an apparatus is e.g. disclosed in the patent US3236240 or in CHEW et al: "A microchannel neuroprosthesis for bladder control after spinal cord injury in rat" (Sci. Transl. Med., vol. 5, no. 210, p. 210ra155, 2013).

It can be that the system comprises an implantable analogue device, a readout circuit, a communication apparatus, a software and an apparatus for unloading fluid from the bladder. In particular the implantable analogue device can comprise the apparatus for unloading fluid from the bladder and can be implanted to the bladder. The readout can be integrated in the communication apparatus on which the software runs. The system can readout the resonant circuit at periodic intervals and warns the user by an optical and/or acoustic signal outputted on the communication apparatus that according to a calibration the storage volume bladder is too high. The user can then use the software or other means to activate the apparatus for unloading fluid from the bladder to unload fluid from the bladder. Such a system is also called a "closed-loop bladder control system" and allows a user with NLUTD to live a fairly conventional bladder-life.

In the following, ways to carry out the invention and embodiments are described referring to drawings. The drawings are all schematical. In the drawings, same reference numerals refer to same or functionally analogous elements. The drawings show:
- Fig. 1: an implantable analogue device comprising of a LC resonant circuit attached to a bladder,
- Fig. 2: a LC resonant circuit with a solenoid coil with a ferrite core,
- Figs 3a - 3d: positioning of implantable analogue device and readout,
- Fig. 4: layout of resonant circuit and readout circuit (frequency sweep),
- Fig. 5: measurement without resonant circuit,
- Fig. 6: measurement with resonant circuit,
- Fig. 7: superposition of measurements with resonant circuits at different strain levels,
- Fig. 8: plot of change of capacitance depending on strain level,
- Fig. 9: layout of resonant circuit and readout circuit (excitation and decay),
- Fig. 10: system comprising a mobile phone,
- Fig. 11: positioning of implantable analogue device attached to or in the bladder wall,
- Figs. 12a-12c: positioning of electrically conductive elements in a support element,
- Figs. 13a-13b: opening and closing trails of electrically conductive elements,
- Fig. 14: manufacturing of an elastically stretchable material.

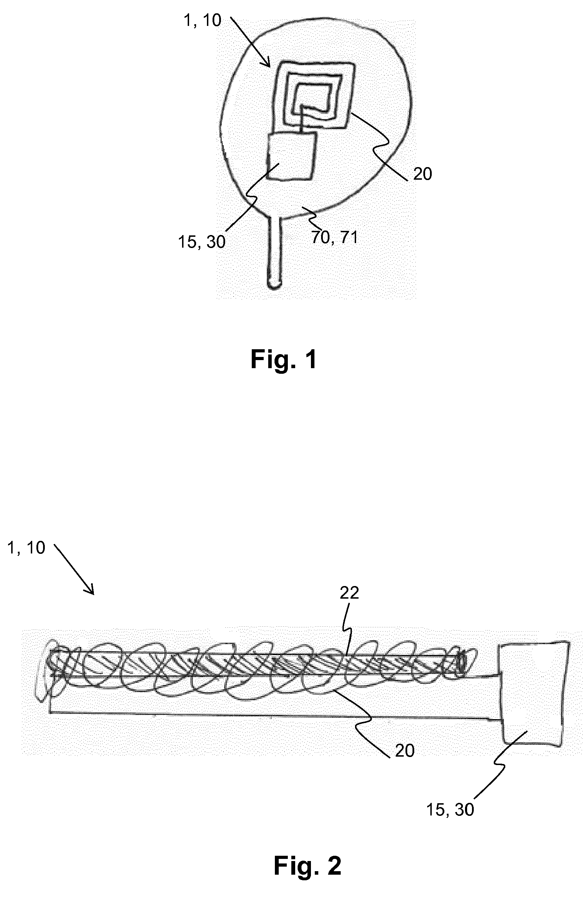

**Figure 1** shows an implantable analogue device 1 attached to the wall of a bladder 70. The attachment can be realized e.g. by gluing or stitching of the implantable analogue device 1 to the outer surface of the bladder wall 71. In a variant, the implantable analogue device 1 is implanted into the bladder wall 71.

The electrically functional part of the implantable analogue device 1 consists of a resonant circuit 10, wherein the resonant circuit 10 consists of a coil 20 and a capacitor 30. At least a part of the resonant circuit 10 comprises a strain sensing section 15. For example the coil 20, the capacitor 30, a part of either or both can be designed as a strain sensing section 15. In the embodiment of Figure 1 the capacitor 30 is designed as a strain sensing section 15. For example, the capacitor 30 can comprise an elastically stretchable material: When the capacitor 30 is stretched, a change in the geometry of the capacitor 30 causes a change in the capacitance of the capacitor 30 which in turn causes a change of the resonant frequency of the resonant circuit 10.

When the bladder 70 changes its volume, strain due to stress is applied to the strain sensing section 15 of the implantable analogue device 1. The resonant circuit 10 changes its resonant frequency depending on the stretch applied to the strain sensing section 15. By reading out the resonant frequency of the resonant circuit 10 it is possible to draw conclusions on the magnitude of strain applied to the strain sensing section 15. The strain (and therefore the resonant frequency) are representative of a physiological parameter of a part of the human or animal body, in this example of the volume resp. the (storage) pressure of the bladder 70.

**Figure 2** shows an embodiment of an implantable analogue device 1 designed as a resonant circuit 10, wherein the coil 20 is designed as a solenoid coil comprising a ferrite core 22. The use of a ferrite core 22 increases the inductance of the coil 20 and therefore allows for a small design of the coil 20, the resonant circuit 10 and/or the implantable analogue device 1.

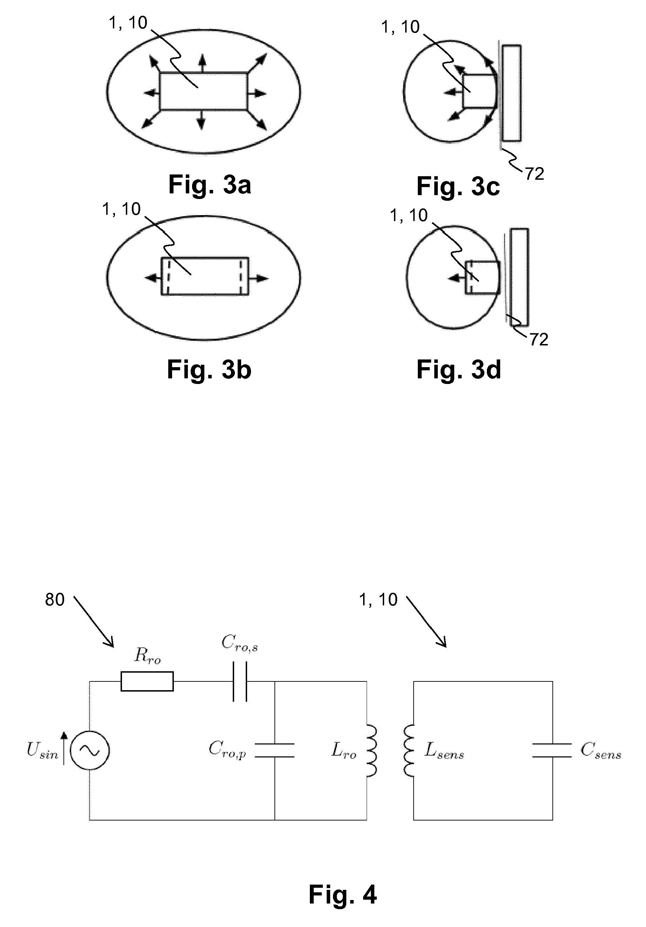

**Figure 3a** shows an implantable analogue device 1 attached to an organ, e.g. the bladder. The implantable analogue device 1 is designed as a multilateral strain sensor. That is to say that the implantable analogue device 1 is designed to determine strain applied in two (or more) dimensions.

**Figure 3b** shows an implantable analogue device 1 designed as a unilateral strain sensor. That is to say that the implantable analogue device 1 is designed to determine strain applied in only one dimension.

A unilateral strain sensor can be suited to easier calibrate the sensor. A multilateral strain sensor can be suited to provide more precise information on the desired physiological parameter.

**Figure 3c** and **Figure 3d** show the positioning of a readout device comprising a readout circuit 80 relatively to the implantable analogue device 1. The readout circuit 80 is designed to determine the resonant frequency of the resonant circuit 10. The implantable analogue device 1 is implanted into a human or animal body. The resonant circuit 10 and the readout circuit 80 are separated by skin 72 and other tissue.

**Figure 4** shows the layout of a resonant circuit 10 and a readout circuit 80, wherein the readout circuit 80 is designed for a readout using a frequency sweep. The readout circuit 80 comprises a sweep generator Uₛᵢₙ feeding a primary inductive coil Lᵣₒ. Lᵣₒ is matched to a reference Line impedance Z₀ (e.g. 50 Ohm) through the components C_{ro,s} und C_{ro,p}. Rᵣₒ represents the source output resistor which is Z₀. A VSWR bridge (not shown) between Rᵣₒ and C_{ro,s} delivers a voltage proportional to the forward travelling wave and a voltage proportional to the reverse travelling wave. The VWSR can be calculated from these two voltages in a microcontroller (not shown).

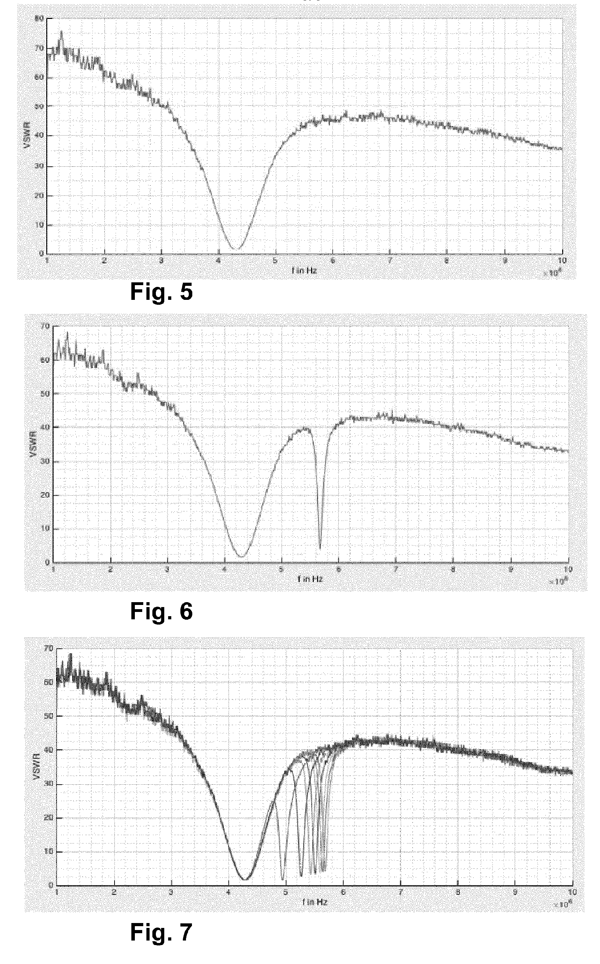

**Figures 5 to 7** show a plot of the Voltage Standing Wave Ratio (abbreviated VSWR) depending on the frequency as a result of a frequency sweep. The chosen frequency band around 5 MHz is just used for an example. Any other frequency might be applicable.

The resonant circuit 10 is normally drawing power from the primary field at its resonance frequency. This leads to less reflected power in the readout device 80 and a better VSWR than without the resonant circuit 10. The resonance frequency of the resonant circuit 10 should be different from the resonance frequency of the primary matched coil in the readout circuit 80. The quality factor of the readout circuit 80 can be not too high, e.g. (3 to 10), and the quality factor of the resonant circuit 10 can be comparatively high, e.g. (10 to 100). The resonant frequency of the resonant circuit 10 can be in the vicinity of the resonance of the readout circuit 80 in order to allow the readout circuit 80 to deliver enough power to the resonant circuit 10 to make a detectable reverse voltage.

**Figure 5** shows the result of a frequency sweep when the readout circuit 80 is not coupled with a resonant circuit 10.

**Figure 6** shows the result of a frequency sweep when the readout circuit 80 is coupled with a resonant circuit 10. The graphical minimum of the VSWR at 5.75 Hz represents the resonant frequency 11 of the resonant circuit 10.

**Figure 7** shows a superposition of measurements using different capacitances in the resonant circuit 10. Different capacities can occur if e.g. capacitor 30 of the resonant circuit 10 comprises a strain sensing section 15. The minima representing the resonant frequency 11 of the resonant circuits 10 vary between 4.94 MHz for a capacitance of 400pF to and 5.68 MHz for a capacitance of 300pF as shown in the following table:

| | | | | | | | |
|---|---|---|---|---|---|---|---|
| Capacitance [pF] | 300 | 305 | 310 | 320 | 330 | 351 | 400 |
| Frequency [MHZ] | 5.68 | 5.64 | 5.60 | 5.51 | 5.43 | 5.26 | 4.94 |

The resonant frequency of a resonant circuit 10 can be a function essentially monotonously depending on the capacitance, in particular an at least approximately reversible function, in particular a reversible linear function.

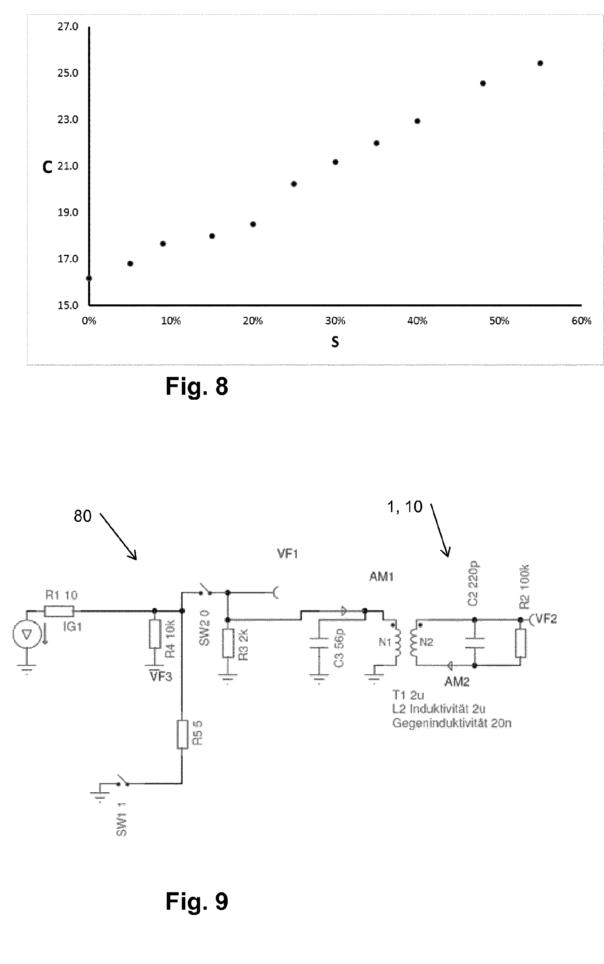

**Figure 8** shows data for the dependency of the capacitance of a capacitor 30 comprising a strain sensing section 15 on the applied strain. In the diagram capacitance (C) in pF is plotted versus (uniaxial) strain (S). 0% strain means that the strain sensing section 15 is not stretched; 100% strain means that the strain sensing section's length (in the chosen direction) is doubled.

The capacitance can be a function essentially monotonously depending on the strain, in particular an at least approximately reversible function, in particular a reversible linear function.

In particular, the resonant frequency of a resonant circuit 10 can be a function essentially monotonously depending on the strain, in particular an at least approximately reversible function, in particular a reversible linear function. Thereby the strain applied to a strain sensing section 15 can - at least approximately - be determined based on the resonant frequency of the resonant circuit 10. The strain can be representative of e.g. volume of a body part, e.g. an organ. A change respectively characteristics and/or variation of a change of the strain of an implantable analogue device 1 attached e.g. to the heart and/or a blood vessel can be representative for various physiological parameters, e.g. the blood pressure and/or the heart rate.

**Figure 9** shows a simplified layout of a resonant circuit 10 and a readout circuit 80, wherein the readout circuit 80 is designed for a readout using excitement and decay. The resonant circuit 10 comprises
- a coil used as secondary inductor (antenna) N2 of a loosely coupled transformer, and
- a capacitor C2.

Resistor R2 fixes the quality factor of the resonant circuit 10. The readout circuit 80 uses a current source IG1 which transmits a carrier frequency of preferably 13.56 MHz (authorized band). The primary part of the loosely coupled transformer comprises antenna coil N1 made resonant at the carrier frequency with capacitor C3. Its quality factor is set by the resistor R3.

The resonant frequency of the resonant circuit 10 is preferably below the resonance frequency of the readout circuit 80.

Switch SW2 (upper) is closed in the beginning of an excitation, while switch SW1 (below) is open. After several microseconds of excitation switch SW1 is closed to shorten the primary circuit in order to empty the energy storage belonging to the exciting frequency (13.56 MHz). Switch SW2 is opened after a further few microseconds to decouple the closed switch SW1 and the excitation source from the primary resonant circuit. The signal at node VF1 can be sensed and analyzed using a Fourier Transformation. In the spectrum two frequency lines are visible, a rest of the excitation frequency and the resonant frequency of the resonant circuit 10 of the implantable analogue device 1.

Alternative implementations using the same principle can be used for the measurement. Optimization of timing and values depend on the selection of the component values and frequencies used.

In one alternative, capacitor C3 is omitted and the primary side of the loosely coupled transformer is not made resonant at all. In another implementation the coil N1 is fed from a voltage source with a parallel output resistor instead of a current source with a series resistance.

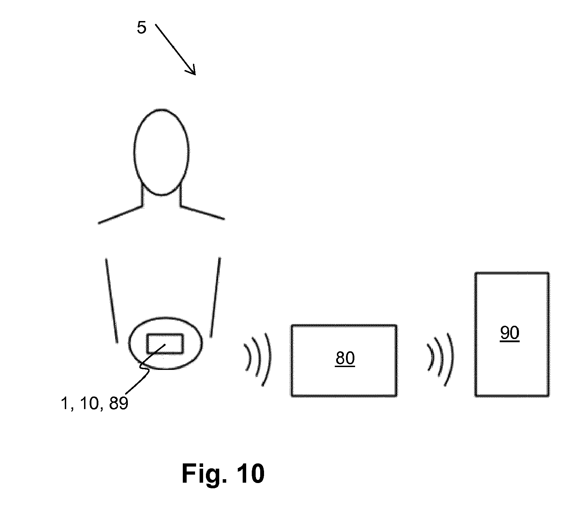

**Figure 10** shows a system 5 comprising an implantable analogue device 1 and a readout circuit 80. The readout circuit 80 can be configured for a readout using a frequency sweep, excitation and decay and/or another method.

The system 5 can further comprise a consumer electric apparatus or communication apparatus 90 such as a mobile phone 91. The readout circuit 80 can communicate with the consumer electric apparatus or a communication apparatus 90 using a wire based connection or a wireless connection. The readout circuit 80 can be integrated in the consumer electric apparatus or communication apparatus 90. For measurement, the readout circuit 80 can be held closely to the implantable analogue device 1, e.g. in a distance of two centimetre or less.

The consumer electric apparatus or communication apparatus 90 can be designed to control the readout and/or other devices, store and/or display the measurement and other information.

The system 5 may comprise further apparatus. For example, if the implantable analogue device 1 and/or the system 5 is designed to monitor the bladder, the system can comprise an implantable apparatus for unloading fluid from the bladder 89. The implantable apparatus for unloading fluid from the bladder 89 can also be controlled using the consumer electric apparatus or communication apparatus 90.

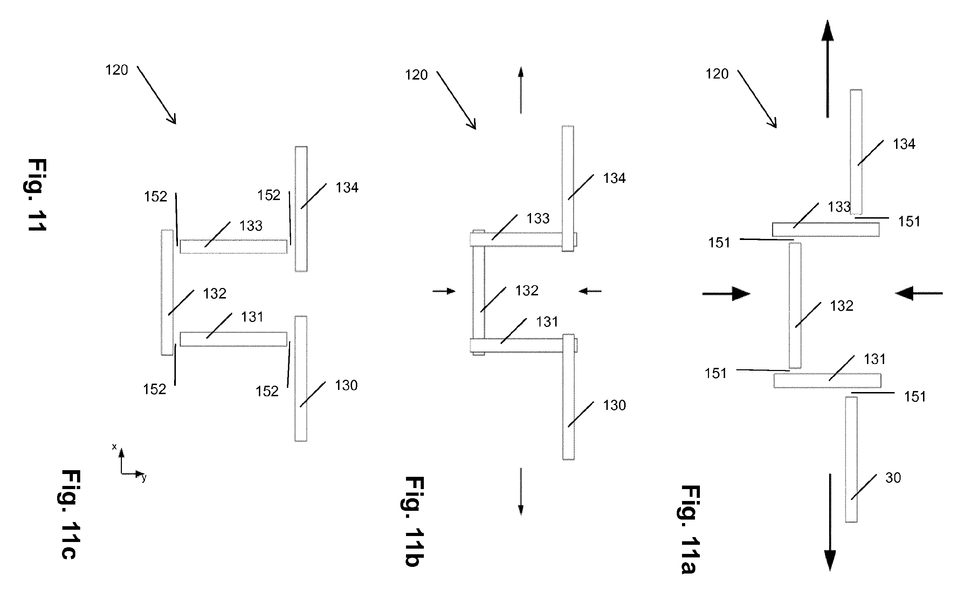

**Figure 11** shows an example of a trail 120 comprising five elongated electrically conductive elements 130, 131, 132, 133, 134 comprised in elastically stretchable material 50 and supported by a support element 51 (not shown in figure 11). The support element 51 is elastically stretchable and electrically insulating. For the description of the figure cylindrical coordinates *(x,y, ϕ)* are chosen, where *x* denotes a chosen direction and y denotes an direction orthogonal to *x*. The directions *x* and *y* are indicated in the figure and *ϕ* is omitted, because the figure only shows a two-dimensional section.

The electrically conductive elements 130, 131, 132, 133, 134 can be grouped in a first group of electrically conductive elements comprising
- a first electrically conductive element 130,
- a third electrically conductive element 132, and
- a fifth electrically conductive element 134
(drawn vertically), and
a second group of electrically conductive elements comprising
- a second electrically conductive element 131, and
- a fourth electrically conductive element 133
(drawn horizontally).

**Figure 11a** shows the trail 120 in a first state of deformation, e.g. an undeformed state. The electrically conductive elements 130, 131, 132, 133, 134 are separated by (horizontal) gaps 152 in the *y*-direction. There is no physical contact between the electrically conductive elements 130, 131, 132, 133, 134 and the conductivity between the electrically conductive elements 130, 131, 132, 133, 134 is low, e.g. zero. Note that also two electrically conductive elements 130, 131, 132, 133, 134 that do not physically touch but are physically close can exchange electric charges, this phenomenon is known as the tunnel-effect. Note that the projections of the electrically conductive elements 130, 131, 132, 133, 134 onto the *x*-coordinate overlap.

**Figure 11b** shows the trail 120 in a second state of deformation resulting from a uniaxial stretching along an axis of the *x*-direction, as indicated by the arrows in the figure. The stretching in the *x*-direction causes a change in the relative position of the projections of the electrically conductive elements 130, 131, 132, 133, 134 onto the *x*-coordinate, but the projections onto the *x*-coordinate still overlap.
Due to the Poisson effect, a stretching in the *x*-direction causes a contraction in the *y-*direction, which in turn causes a change in the projections of the electrically conductive elements 130, 131, 132, 133, 134 onto the *y*-coordinate. In figure 11b this has caused the gaps 152 in the *y*-direction to close: the projections of the electrically conductive elements 130, 131, 132, 133, 134 on the *y*-coordinate now overlap.
The electrically conductive elements 130, 131, 132, 133, 134 now physically touch, and the trail 120 can conduct current. In particular the electrically conductive elements 130, 131, 132, 133, 134 of the two groups of electrically conductive elements are connected.

**Figure 11c** shows the trail 120 in a third state of deformation resulting from a further uniaxial stretching along the same axis as before. The further stretching in the *x-*direction causes a further change in the projections of the electrically conductive elements 130, 131, 132, 133, 134 onto the *x*-coordinate and now the projections onto the *x*-coordinate do no longer overlap. The electrically conductive elements 130, 131, 132, 133, 134 are now separated by (vertical) gaps 151 in the *x*-direction.
There is no longer a physical contact between the electrically conductive elements 130, 131, 132, 133, 134 and the conductivity between the electrically conductive elements 130, 131, 132, 133, 134 is again low, e.g. zero.
The conductivity function of the trail for the described uniaxial stretching is a step function: at first zero (or almost zero), then constant (or almost constant) at a higher level, and then again zero (or almost zero). By superimposing several step functions every conductivity function of practical interest can be approximated. In practice the superimposition can be realized e.g. by connecting various trails 120 in parallel.

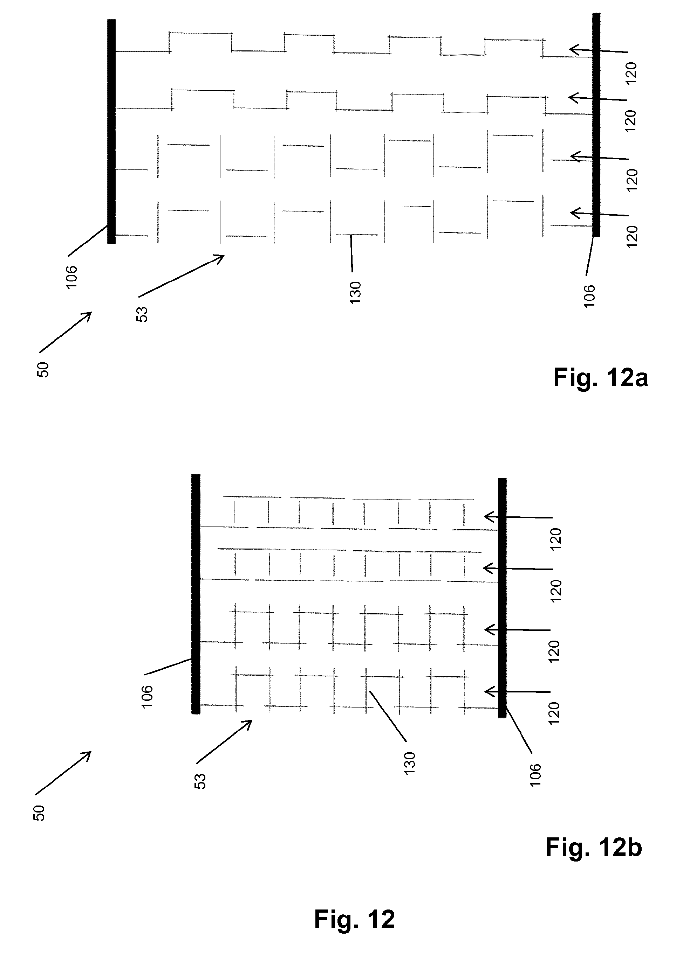

**Figure 12** schematically shows an example of a track 53 connecting a first and a second terminal 106 in an elastically stretchable material 50. The track 53 comprises four trails 120 comprising elongated electrically conductive elements 130. Each trail 120 comprises electrically conductive elements 130 arranged similarly to those in the embodiment of figure resonant frequency 11, but other arrangements are possible as well. The support element 51 is omitted from the figure for clarity.

**Figure 12a** shows the track 53 in a first state of deformation, e.g. an undeformed state. The electrically conductive elements 130 of the left two trails 120 are physically and electrically connected and electrically connect the first and the second terminal 106. The electrically conductive elements 130 of the right two trails 120 are physically not connected. The first and the second terminal 106 are electrically connected by the two left trails 120.

**Figure 12b** shows the track 53 in a second state of deformation, e.g. when uniaxial strain is applied. Now the electrically conductive elements 130 of the left two trails 120 are no longer physically connected. Instead, now the electrically conductive elements 130 of the right two trails 120 are physically connected. Thus the first and the second terminal 106 are electrically connected by two trails 120.

Assume the left two trails 120 disconnect at exactly the amount of stretch at which the right two trails 120 connect. Then the first and the second terminal 106 are physically connected by two trails for all states of deformation between the embodiments shown in figure 12a and figure 12b. The electrical conductivity between the first and the second terminal 106 at every stage (between the stage of figure 12a and that of figure 12b) is realized by two trails 120 physically connecting the first and the second terminal 106; and the conductivity between the first and the second terminal 106 is constant (or almost constant) for all uniaxial strain values between that of figure 12a and figure 12b.

The embodiment shown figure 12 can be used for e.g. creating an electrically stretchable material 50, wherein the elastically stretchable material 50 is - in at least one direction - elastically stretchable at least up to 100% strain, and wherein the elastically stretchable material 50 is - at least partially - electrically conductive for any strain in the at least one direction at least up to 100% strain.

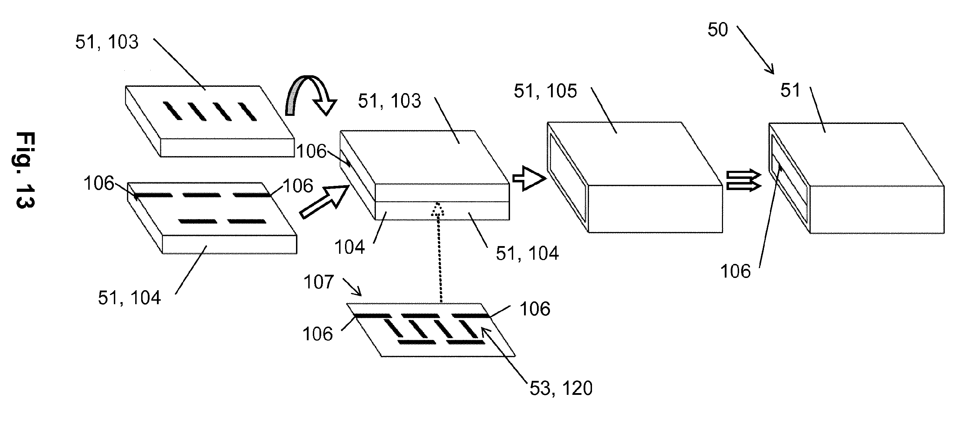

**Figure 13** schematically shows a method of producing an elastically stretchable material 50 comprising
- a support element 51 that is elastically stretchable and insulating, and
- electrically conductive elements 130 supported by the support element 51 and configured to - for any strain at least up to 100% strain in the at least one direction
- form electrically conductive tracks 53.

In this example the support element 51 comprises three components 103, 104, 105 made of elastically stretchable and insulating material; namely an upper component 103, a lower component 104 and a sleeve 105. The track 53 of the embodiment comprises a single trail 120 comprising of a first and a second group of electrically conductive elements. The electrically conductive elements of the first group of electrically conductive elements are arranged to be located at a lower side of the upper component 103. The electrically conductive elements of the second group of electrically conductive elements are arranged to be located at an upper side of the lower component 104. The electrically conductive elements of the two groups can be manufactured and/or arranged on the respective component by e.g. photolithographic and/or other processes.

In a next step, the upper component 103 and the lower component 104 are put together so that the lower side of the upper component 103 and the upper side of the lower component 104 touch. Thereby the electrically conductive elements of the upper component 103 and of the lower component 104 are located adjacent to a common plane 107. In the example shown in figure 13 the electrically conductive elements of the trail are initially not physically connected. In a further step, the two closely spaced components 103, 104 are inserted into the sleeve 105, which preferably is tight enough to press together and keep together the two closely spaced components 103, 104.

The final composite structure forming the elastically stretchable material (50) is shown on the right side. Two terminals 106, one on a visible side and one on a not visible side, allow for electrical connection to e.g. non-strain sensitive parts of a circuit. According to the arrangement of the two groups in the common plane 107, the electrically conductive elements of the trail 120 are not connected in an undeformed state. If a certain level of strain is applied, the electrically conductive elements are compressed due to the Poisson effect and the gaps in the common plane 107 close so that the electrically conductive elements of the two groups of electrically conductive elements physically and electronically connect. By designing a multitude of different trails 120 with a certain opening and closing behaviour - by superposition - almost any function of conductivity in dependence of on applied strain can be constructed.

## Claims

1. Implantable analogue device (1)
- configured for determining parameters representative of physiological parameters of a part of a human or animal body, and
- configured for wireless transmission of the determined parameters,
wherein the implantable device (1) comprises a resonant circuit (10) comprising a coil (20) and a capacitor (30), and
wherein the resonant circuit (10) comprises a strain sensing section (15), and wherein the resonant circuit (10) is configured to change its resonant frequency depending on a strain applied to the strain sensing section (15).

2. Implantable analogue device (1) according to claim 1,
wherein the implantable analogue device (1) is
- configured for determining parameters representative of physiological parameters of a part of a human or animal body,
and is
- configured for wireless transmission of the determined parameters.

3. Implantable analogue device (1) according to one of the preceding claims,
wherein the resonant circuit (10) comprises two or more strain sensing sections (15).

4. Implantable analogue device (1) according to one of the preceding claims,
wherein - at least for an interval of strain applied in at least one direction - is a function essentially monotonously depending on a uniaxial strain applied to the strain sensing section (15),
in particular wherein the interval is between 0% uniaxial strain and 100% uniaxial strain in the at least one direction.

5. Implantable analogue device (1) according to one of the preceding claims,
wherein the coil is configured to change its inductance depending on a strain applied to the coil (20) or parts thereof; and
wherein the resonant circuit (10) is configured to change its resonant frequency depending on the change of the inductance of the coil (20).

6. Implantable analogue device (1) according to one of the preceding claims,
wherein the capacitor is configured to change its capacitance depending on a strain applied to the capacitor (30) or parts thereof; and
wherein the resonant circuit is (10) configured to change its resonant frequency depending on the change of the capacitance of the capacitor (30).

7. Implantable analogue device (1) according to one of the preceding claims,
wherein the electrically functional parts of the implantable analogue device (1) form the resonant circuit (10),
in particular wherein the resonant circuit (10) consists solely of the coil (20) and the capacitor (30).

8. Implantable analogue device (1) according to one of the preceding claims, wherein the resonant circuit (10) further comprises a resistor (40),
wherein the resistor (40) is configured to change its impedance depending on a strain applied to the resistor or parts thereof, and
wherein the resonant circuit (10) is configured to change its quality factor depending on a change of the impedance of the resistor (40).

9. Implantable analogue device (1) according to one of the preceding claims, wherein the strain sensing section (15) comprises an elastically stretchable material (50), wherein the elastically stretchable material (50) is - in at least one direction - elastically stretchable at least up to 10% strain, and wherein the elastically stretchable material (50) is - at least partially - electrically conductive for any strain in the at least one direction at least up to 10% strain.

10. Implantable analogue device (1) according to one of the preceding claims, wherein the strain sensing section (15) comprises an elastically stretchable material (50), wherein the elastically stretchable material (50) is - in at least one direction - elastically stretchable at least up to 100% strain, and wherein the elastically stretchable material (50) is - at least partially - electrically conductive for any strain in the at least one direction at least up to 100% strain.

11. Implantable analogue device (1) according to one of claims 9 to 10,
wherein the elastically stretchable material (50) comprises
- a support element (51) that is elastically stretchable and electrically insulating, and
- electrically conductive elements (130) supported by the support element (51) and configured to - for any strain at least up to 100% strain in the at least one direction of strain - form electrically conductive tracks (53),
▪ in particular wherein the electrically conductive elements (130) are particles, wires, ribbons and/or sheets, and/or
▪ in particular wherein at least some of the electrically conductive elements (130) in at least one dimension are smaller than 10µm, and /or
▪ in particular wherein the electrically conductive elements (130) form a dynamic network (55) of connections,
• in particular wherein the network (55) of connections is configured so that that the number and topology of the connections depends on the applied strain.

12. Implantable analogue device (1) according to one of claims 9 to 11,
wherein
- the coil (20) or a part of the coil (20), and/or
- the capacitor (30) or a part of the capacitor (30)
are made of the elastically stretchable material (50).

13. Implantable analogue device (1) according to one of claims 9 to 12,
wherein the resonant circuit (10) is completely made of the elastically stretchable material (50),
in particular wherein the implantable analogue device (1) is completely made of the elastically stretchable material (50).

14. Implantable analogue device (1) according to claim 7 and one of claim 9 to 12,
wherein
- either only the coil (20)
- or only the capacitor (30)
is made of the elastically stretchable material (50).

15. Implantable analogue device (1) according to one of the preceding claims,
wherein the coil comprises a ferrite core (22).

16. Implantable analogue device (1) according to one of the preceding claims,
wherein the coil (20) is a planar coil, a meander coil, a foil coil and/or a ribbon coil,
in particular wherein the diameter of the coil (20) is less than 3 cm.

17. Implantable analogue device (1) according to one of the preceding claims,
wherein the coil (20) is a solenoid coil or a spring coil,
in particular wherein the length of the coil is less than 2cm and the diameter of the profile of the coil (20) is less than 5 mm.

18. Implantable analogue device (1) according to one of the preceding claims,
wherein the inductance of the coil (20) is between 100 nH and 1 mH.

19. Implantable analogue device (1) according to one of the preceding claims,
wherein the capacitor (30) is a radial capacitor, a plate capacitor and/or a finger capacitor,
in particular wherein the capacitor (30) is less than 1 cm wide and less than 2 cm long.

20. Implantable analogue device (1) according to one of the preceding claims, wherein the capacitance of the capacitor (30) is between 100 fF and 1nF.

21. Implantable analogue device (1) according to one of the preceding claims, wherein the resonant circuit (10) comprises two or more capacitors (30),
- wherein a first capacitor (30) has a fixed capacitance, and
- a second capacitor (30) has a capacitance depending on the applied strain,
in particular wherein the first capacitor (30) is un-stretchable.

22. Implantable analogue device (1) according to one of the preceding claims,
wherein - if the implantable device (1) is implanted to or into a bladder wall (71) - the implantable analogue device (1) is configured to measure
- a volume of the bladder (70), and/or
- an extension of the bladder (70), and/or
- a pressure of the bladder (70) and/or
- a physiological parameter related to the volume, the extension and/or the pressure of the bladder (70).

23. System (5) comprising
- an implantable analogue device (1) according to one of the preceding claims; and
- a readout circuit (10) for reading out the resonant frequency of the resonant circuit (10) of the implantable analogue device (1).

24. System (5) according to claim 23,
wherein the readout circuit (80) is configured for a readout using a frequency sweep,
namely
- the readout circuit (80) emits a sweep signal in a pre-defined frequency band,
- the readout circuit (80) measures the reflected voltage and/or standing wave ratio for each frequency in the sweep signal,
- the readout circuit (80) determines the resonant frequency of the resonant circuit (10) and/or the coupled resonant circuit (10) and readout circuit (80) based on local extreme points of the measured voltages and/or standing wave ratio.

25. System (5) according to claim 23,
wherein the readout circuit (80) is configured for a readout using excitation and decay, namely
- the readout circuit (80) induces a current of a chosen frequency into the resonant circuit (80), thereby storing energy in the resonant circuit (10), and
- the readout circuit (80) stops inducing the current, and
- the readout circuit (80) measures the oscillation frequency of the resonant circuit (10).

26. System (5) according to claims 23 to 25,
comprising two or more implantable analogue devices (1),
in particular wherein resonant frequency of the resonant circuit (10) of a first implantable analogue device (1) is substantially different from the resonant frequency of the resonant circuit (10) of a second implantable analogue device (1).

27. System (5) according to one of the claims 23 to 26,
comprising a communication apparatus or a consumer electronic apparatus (90)
in particular wherein the readout circuit (80) is integrated in the communication apparatus or a consumer electronic apparatus (90).

28. System (5) according to claim 27, further comprising a software,
wherein the software
- provides a graphical user interface (GUI) for patients and/or doctors, and/or
- is configured for data analysis, and/or
- is configured for graphical display of data.

29. System (5) according to one of the claims 23 to 28,
comprising an apparatus for unloading fluid from the bladder (89).
